# EUROPÄISCHE PATENTSCHRIFT

(11) **EP 2 611 394 B1**
(45) Veröffentlichungstag und Bekanntmachung des Hinweises auf die Patenterteilung: **23.08.2017**
(21) Anmeldenummer: 11801569.2
(22) Anmeldetag: 01.09.2011
(51) Int. Cl.: A61F 2/28, A61F 2/30, A61F 2/44, A61B 17/80, A61B 17/86

(54) **KNOCHENVERANKERUNGS- ODER VERBINDUNGSEINRICHTUNG DIE EINEN DEHNUNGSREIZ INDUZIERT**
BONE-ANCHORING OR BONE-CONNECTING DEVICE THAT INDUCES AN ELONGATION STIMULUS
DISPOSITIF D'ANCRAGE D'OS OU DE LIAISON D'OS QUI INDUIT UN STIMULUS D'EXTENSION

(30) Priorität: 03.09.2010 DE 102010040228
(43) Veröffentlichungstag der Anmeldung: 10.07.2013
(73) Patentinhaber: Aces GmbH, 70794 Filderstadt (DE)
(72) Erfinder: HEUER, Frank, 70794 Filderstadt (DE); TRAUTWEIN, Frank, 70794 Filderstadt (DE); FRANKE, Jörg, 39118 Magdeburg (DE); PUTZIER, Michael, 14532 Stahnsdorf (DE); KOTHE, Ralph, 22397 Hamburg (DE); MATGÉ, Guy, L-8229 Mamer (LU); LILJENQVIST, Ulf, 48147 Münster (DE)
(74) Vertreter: Patentanwälte Magenbauer & Kollegen Partnerschaft mbB
(86) Internationale Anmeldenummer: PCT/DE2011/050029
(87) Internationale Veröffentlichungsnummer: WO 2012/045307

(56) Entgegenhaltungen:
- EP-A2- 0 950 389
- DE-U1- 20 015 265

## Beschreibung

### Technisches Gebiet

Die Erfindung betrifft ein Implantat in Form einer Knochenverankerungs- oder Verbindungseinrichtung das vorzugsweise zur Stabilisierung der Wirbelsäule implantiert werden kann. Das Implantat kann eine hohe Eigensteifigkeit besitzen, welche eine Dimensionierung und Auslegung auf hohe Ermüdungsfestigkeit und Implantathaltbarkeit erlaubt. Durch die erfindungsgemäße Ausbildung und Anordnung von Oberflächenstrukturen auf einem Strukturträger kann - trotz hoher Implantatsteifigkeit - ein physiologisch wirksamer Dehnungsreiz entlang der Implantatoberfläche bzw. zwischen einzelnen Elementen innerhalb des Implantats generiert werden. Die Oberflächenelemente sind derart dimensioniert und auf die Belastung sowie Steifigkeit des Implantats abgestimmt dass ein physiologischer Dehnungsreiz, das heißt ein Dehnungsreiz der das Knochenwachstum anregt, erzeugt wird.

### Stand der Technik

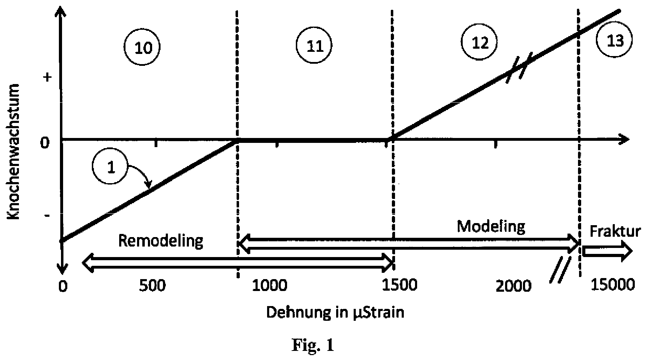

Für die chirurgische Behandlung, insbesondere bei einer Osteosynthese oder Stabilisierung von Wirbelsäulenerkrankungen ist eine Vielzahl von Implantaten mit schraubbaren Verankerungseinrichtungen (z.B. "Pedikelschrauben") bekannt. Oft wird das Problem der Schraubenlockerung beschrieben, welches eine Folge des sogenannten "Stress-Shieldings" ist. Der Effekt des "Stress-Shieldings" geht auf die biologischbiomechanischen Grundlagen der mechanischen Stimulation des Knochengewebes zurück (Ignatius, 2005; Baas, 2010). Ein funktioneller Zusammenhang (1) wurde von Frost in einem Review beschrieben (Frost 1987) (Fig. 1). Wird beispielsweise eine Dehnung von weniger als 800 *µ*Strain (entspricht 0,08%) am Knochengewebe appliziert, findet das sogenannte "Remodeling" (10) des Knochens statt. Beim "Remodeling" wird Knochen repariert und umgebaut, wobei die Knochenmasse und Knochenfestigkeit jedoch tendenziell abnimmt. Ab einer Dehnung von 800 bis 1500 *µ*Strain existiert ein Gleichgewichtszustand zwischen dem "Remodeling" und dem "Modeling" (11). Hier wird Knochen gleichermaßen abgebaut und wieder aufgebaut, die Knochenmasse und Knochenfestigkeit bleibt unverändert. Ab einer Dehnung von 1500 *µ*Strain findet vorwiegend der Knochenaufbau ("Modeling") statt, welcher bei ca. 2000 *µ*Strain sein Maximum findet (12). Wird die Dehnung weiter erhöht, wird der Knochen nekrotisch bzw. kann er seine strukturelle Integrität verlieren. Ab ca. 15000 *µ*Strain bricht Knochen (13). Somit besitzt der typische Knochen, beispielsweise die Tibia, einen Sicherheitsfaktor von etwa 5 bis 7 zwischen maximal typischer Verformung (maximal 2000 bis 3000 *µ*Strain) und seiner Bruchgrenze (ca. 15000 *µ*Strain). Wird nun eine Schraube in den Knochen platziert, so existieren strukturelle Unterschiede. Eine aus einer Titanlegierung gefertigte Schraube besitzt einen E-Modul von ca. 105 GPa. Knochen besitzt ein E-Modul von ca. 22 GPa an der Kortikalis und <1 GPa an der Spongiosa (Lu, 1996). Aufgrund dieser Unterschiede stabilisiert die vielfach steifere Schraube das umgebende Knochengewebe so, dass sie einen natürlichen Dehnungsreiz vom Knochengewebe abschirmt und den negativen "Remodeling" Prozess in der Nähe der Schraube begünstigt. Eine abnehmende Knochensubstanz bzw. Festigkeit provoziert damit Schraubenlockerungen, welche oftmals einer zusätzlichen Revisions-Operation bedürfen.

Eine generelle Methode das Knochenwachstum mit einem Dehnungsreiz anzuregen wurde in WO9324092A1 gezeigt. Hier basiert die Methode jedoch auf einer systematischen, äußeren Belastung die auf alle Knochen unter Ausnutzung der Schwerkraft bzw. Massenträgheit gleichzeitig einwirkt.

Aus EP1430846B1 ist bekannt, dass mit Vergrößerung der Schraubenoberfläche und einer damit geschaffenen Porosität das Knochenwachstum begünstigt werden kann. Ein Nachteil dieses Aufbaus ist der erheblich reduzierte Querschnitt, die Erzeugung von Spannungsspitzen im Bereich der Bohrungen und damit die reduzierte Ermüdungsfestigkeit des Knochenankers.

Aus WO2004017857A1 ist eine Methode bekannt, mit welcher Verankerungseinrichtungen in den Knochen platziert und anschließend mit Ultraschall beaufschlagt werden um ein Kunststoffmaterial durch Temperaturerhöhung zu verflüssigen, welches dann in die Knochenzwischenräume fließt und damit eine mechanische Verbindung eingeht. Das dort beschriebene Material ist jedoch verhältnismäßig weich, und damit für eine Osteosynthese oder lasttragende Implantate eher ungeeignet ist. Ferner ist durch die Temperatureinwirkung die Gefahr einer lokalen Schädigung des Gewebes bzw. Knochens gegeben.

In WO0032125A1 wurde eine Gewindeform vorgestellt, mit welcher beim Einschrauben der Knochen komprimiert wird. Dies erhöht die Primärstabilität, jedoch unterliegt diese Anordnung in gleicher Weise dem eingangs beschriebenen "Stress-Shielding".

Aus DE908906 ist eine federnde Knochenschraube bekannt die Knochenfragmente mit einer dauerhaften Vorspannung gegeneinander pressen soll. Hierdurch wird zwar das "Stress-Shielding" weitgehend vermieden, allerdings darf angezweifelt werden ob eine derartige Schraube die notwendige Haltbarkeit zur Stabilisierung von Knochen aufweist.

Die DE 20015265 U1 offenbart ein zylindrisches Implantat aus Metall zum Einsetzen zwischen zwei Wirbelkörper in die Wirbelsäule mit einem an die Wirbelkörper-Endflächen angepassten Querschnitt mit einer oder mehreren durch das Implantat in Richtung der Implantatachse hindurchgehenden Öffnungen zum Einsprossen von Knochen, wobei in das Implantat senkrecht zur Achse von zwei, drei oder vier Richtungen je ein oder mehrere, übereinander liegende Einschnitte eingebracht sind, die zu einer dem Knochen ähnlichen Flexibilität des Implantats in Richtung der Implantatachse führen, die eine Übertragung eines physiologischen, mechanischen Wachstumsreizes auf die in die Öffnungen einsprossenden Zellen bei funktioneller Wechselbelastung erlaubt, so dass sich der für die Fusion der Wirbelkörper erforderliche Knochen durch die Öffnungen des Implantats hindurch bildet und erhalten bleibt.

Die EP 0950389 A2 offenbart ein Zwischenwirbelfusionsimplantat mit zwei Stützkörpern aus einem Material, dessen Steifigkeit grösser ist als die des Knochenmaterials von Wirbelkörpern, von denen jeder eine Stützfläche zur Anlage an einem von zwei benachbarten Wirbelkörpern aufweist, deren Fläche kleiner ist als die Querschnittsfläche der Wirbelkörper, mit einer Führung, durch die die beiden Stützkörper quer zu den Stützflächen unverkippbar gegeneinander verschiebbar geführt sind, und mit einem Federelement, das die beiden Stützkörper längs der Führung auseinanderschiebt.

Des Weiteren existieren einige Varianten von Schraubensystemen bei denen Knochenzement injiziert wird um die Schraube im geschwächten Knochen (z.B. bei Osteoporose) zu augmentieren. Die Zementinjektion ist irreversibel und birgt zahlreiche Risiken (unerwünschte Zementaustritte in Spinalkanal, Bandscheibe oder das Gefäßsystem, Nekrotisierung durch hohe lokale Reaktionstemperaturen, Korrosion zwischen Implantat und Röntgenkontrastmittelbeimengung).

Aus Reigstad, 2007 ist bekannt, dass die Beschichtung von Schrauben das Knochenwachstum nach einer kurzen Einheilungszeit von 12 Wochen erhöht. Die zusätzliche Beschichtung mit (Tri)Kalziumphosphat und Hydroxylapatit zeigt einige Erfolge. Die nur wenige Mikrometer dicke Schicht wird jedoch nach der Einheilungsphase komplett resorbiert (Reigstad, 2007). An dieser Stelle fehlen die Langzeitbeobachtungen, die ein Knochenabbau ("Remodeling") nach der Schichtresorption ausschließen könnten. Zu erwarten wäre eine Knochenabnahme an der beschichteten Schraube nachdem die Beschichtung resorbiert wurde. Die Beschichtung verursacht, vom fraglichen Langzeitnutzen abgesehen, deutliche Mehrkosten in der Produktion derartiger Knochenanker. Zudem sind die mit dem Zulassungsprozess verbundenen Kosten eines durch die Beschichtung "aktiven" Implantats signifikant höher im Vergleich zu einem unbeschichteten Implantat.

In der Vergangenheit wurden mehrere federelastische bzw. dynamische Implantate vorgestellt, welche aufgrund einer reduzierten Eigensteifigkeit höhere Dehnungen (Mikrobewegungen) auf den Knochen erlauben (DE10348329B3, 2003; EP1943986A2, 2005). Diese Dehnung soll so das Knochenwachstum stimulieren. Diese Ansätze haben jedoch die Nachteile einer reduzierten Ermüdungsfestigkeit und einer hohen Variabilität der Dehnung in Abhängigkeit von der Belastung, die leicht zu einer Dehnung weit über dem für den Knochenaufbau idealen Dehnungsreiz führen kann.

Ein weiteres klinisches Phänomen ist das Ausbleiben der Knochenheilung bei einem Knochendefekt, der eine bestimmte Größe überschritten hat ("Critical Size Defect"). Hier bleibt der Dehnungsreiz zwischen den Knochenfragmenten aus. Aus der klinischen Praxis ist bekannt, dass verschiedene Knochenersatzmaterialen eingesetzt werden um den Knochenspalt zu überbrücken. Jedoch sind die meisten Knochenersatzmaterialen dahin gehend limitiert, dass sie den Belastungen nicht standhalten bzw. mechanisch ungünstig degradieren und den lokalen Säurespiegel anheben (Sarkar, 2006), was insbesondere bei Implantaten mit reduzierter Eigensteifigkeit von Nachteil ist.

Ein steifes Implantat zur Verringerung der Knochendefektgröße mit gleichzeitig kontrollierter mechanischer Dehnungsreiz-Stimulation über die Fusionsstrecke ist nicht bekannt.

### Darstellung der Erfindung

### Technische Aufgabe

Die Aufgabe der vorliegenden Erfindung besteht in der Bereitstellung eines Implantats das sich zur Lastübertragung mit dem Knochen verbinden lässt und durch eine Relativbewegung von geeigneten, mit dem Implantat verbundenen Strukturen einen physiologischen Dehnungsreiz auf das Gewebe ausübt, mit dem Ziel die knöcherne Einheilung zu verbessern bzw. zu beschleunigen.

### Technische Lösung

Die Aufgabe wird dadurch gelöst, dass lamellenartige Oberflächenelemente oder Strukturen (nachfolgend auch Strukturträger genannt) an der Oberfläche eines Implantatträgers angeordnet sind. Die Strukturträger sind vom Implantatträger weitgehend entkoppelt, aber an wenigstens einer Stelle mit dem Implantatträger, insbesondere im Bereich der Grenzflächen zur Anbindung an den vorhandenen Knochen, verbunden.

Dementsprechend betrifft die Erfindung ein Implantat in Form einer Knochenverankerungs- oder Verbindungseinrichtung bestehend aus einem Implantatträger, an dem mindestens ein Grenzflächenpaar zur Krafteinleitung und Anbindung an eine knöcherne Struktur ausgebildet ist, wobei mindestens zwei Strukturträger vorgesehen sind, an denen jeweils Strukturelemente ausgebildet sind, wobei jeder der Grenzflächen mindestens ein Strukturträger zugeordnet ist, dass mindestens Teilbereiche der Strukturträger ineinander greifen oder überlappen und dass eine lokale Relativbewegung der Strukturträger zueinander in etwa einer bei Belastung der Grenzflächen auftretenden Relativbewegung der Grenzflächen entspricht.

Bei einer Weiterbildung der Erfindung ist vorgesehen, dass die Strukturträger eine Vielzahl kleinerer, nicht vollständig abgeschlossener Bereiche definieren, die bei Implantatbelastung verformt werden.

Bei einer Weiterbildung der Erfindung ist vorgesehen, dass eine Teilung der Strukturelemente auf den Strukturträgern und/oder ein Abstand zwischen den Strukturträgern und eine Steifigkeit des Implantatträgers derart auf eine erwartete mechanische Belastung des Implantats abgestimmt sind, dass die nicht vollständig abgeschlossenen Bereiche bei einer Implantatbelastung eine Dehnung in einem für den Knochenaufbau günstigen Bereich erfahren.

Bei einer Weiterbildung der Erfindung ist vorgesehen, dass die Strukturelemente selbsttragend ausgebildet sind.

Bei einer Weiterbildung der Erfindung ist vorgesehen, dass die die Strukturelemente an den Strukturträgern als Lamellen, Gitter, Ausformungen, Zähne, Ausnehmungen, Bohrungen, Poren, Texturen, Aufrauhungen, Beschichtungen oder Kavitäten ausgebildet sind.

Bei einer Weiterbildung der Erfindung ist vorgesehen, dass eine Oberfläche der Strukturträger mit Strukturelementen versehen ist, die eine Strukturgröße im Bereich von 0.2*µ*m bis 100*µ*m aufweisen.

Bei einer Weiterbildung der Erfindung ist vorgesehen, dass die Strukturträger an einer der Grenzflächen mit einem Bereich mit niederer Steifigkeit und an einer anderen Grenzfläche mit einem Bereich mit höherer Steifigkeit angebunden sind.

Bei einer Weiterbildung der Erfindung ist vorgesehen, dass die einander zugewandten Strukturträger abwechselnd mit je einer der Grenzflächen verbunden sind.

Bei einer Weiterbildung der Erfindung ist vorgesehen, dass die Strukturträger mit einer Substanz zur Beschleunigung von Knochenbildung beschichtet oder angereichert sind und/oder dass die Bereiche zwischen den Strukturträgern mit einer Substanz zur Beschleunigung von Knochenbildung gefüllt sind.

Bei einer Weiterbildung der Erfindung ist vorgesehen, dass Dehnungen in den Bereichen in Folge der Implantatbelastung zwischen 100 und 15.000 *µ*m/m, und in der Einheilungsphase insbesondere zwischen 500 und 10.000 *µ*m/m liegen.

Bei einer Weiterbildung der Erfindung ist vorgesehen, dass die maximale Relativbewegung der Strukturträger durch eine oder mehrere Wegbegrenzungen limitiert ist.

Bei einer Weiterbildung der Erfindung ist vorgesehen, dass die maximale Relativbewegung der Strukturträger auf Werte zwischen 0,1*µ*m und 200*µ*m beschränkt ist.

In einer bevorzugten Ausführung sind mehrere Strukturträger jeweils abwechselnd an gegenüberliegenden Seiten der Grenzflächen des Implantatträgers angebunden. Die Strukturträger können außerdem Vertiefungen bzw. Öffnungen (Poren) enthalten, in die sich Knochenzellen verankern können. Wird das Implantat durch den Patienten belastet, führen die Strukturträger Relativbewegungen aus. Die Steifigkeit des Implantatträgers und die Teilung bzw. der Zwischenraum der Strukturträger sind sowie deren maximal möglichen Verschiebung ist so ausgelegt, dass der durch die Strukturträger auf das Gewebe ausgeübte Dehnungsreiz zur (beschleunigten) Bildung von Knochenzellen führt. Die erfindungsgemäße Anordnung der Strukturträger und Strukturen auf dem Strukturträger kann in Knochenverankerungseinrichtungen und Knochenüberbrückungselementen gleichermaßen eingesetzt werden und verfolgt jeweils das Ziel der Stimulierung des Knochenwachstums im Bereich des Implantats.

### Vorteilhafte Wirkungen

Vorteilhaft bei der vorliegenden Erfindung ist, dass das erfindungsgemäße Implantat einen Dehnungsreiz auf das umgebende Knochengewebe ausübt und den "Modeling"-Prozess induziert. Dies erhöht die Verankerungsfestigkeit mit dem Knochen und reduziert somit Implantatlockerungen. Ein großer Vorteil ist, dass die relativ hohen, physiologisch wirksamen Dehnungen an der Oberfläche mit einem Implantat generiert werden können, welches gleichzeitig eine hohe Eigensteifigkeit besitzt. Das Problem der ausbleibenden knöchernen Überbrückung bei großen Defektstellen aufgrund des fehlenden Dehnungsreizes wird von der Erfindung gelöst. Des Weiteren ist die Anordnung der Strukturträger kaum ausschlaggebend für die Dauerfestigkeit des Implantats, da sie vom Lasttragenden Implantatträger entkoppelt sind. Die Anordnung der Strukturträger bzw. deren Strukturen erlaubt es verschiedene Dehnungsbereiche zu bilden. Es können beispielsweise lineare Dehnungsbereiche mit nicht-linearen Bereichen oder mit Bereichen höherer Dehnungen kombiniert werden. Durch die mechanische Knochenwachstumsstimulation stellt die vorliegende Erfindung eine Alternative zu Implantatbeschichtungen und der Anwendung von teuren biologischen Präparaten zur Stimulierung des Knochenwachstums dar. Neben höherer Implantat- und Verankerungsstabilität und höheren Fusionsraten mit besserer Knochenqualität sind Kostenvorteile durch das erfindungsgemäße Implantat realisierbar.

### Kurze Beschreibung der Zeichnungen

Fig. 1 zeigt den funktionellen Zusammenhang aus dem Knochenwachstum und der applizierten Dehnung im Knochengewebe.
Fig. 2 erläutert die Erzeugung der lokalen Dehnung an der Implantatoberfläche.
Fig. 3 zeigt verschiedene Anordnungen der Oberflächenlamellen.
Fig. 4 stellt ein Beispiel eines Knochenüberbrückungselements vor.
Fig. 5 zeigt die Verwendung von einzelnen Oberflächenlamellen an einem Implantat.
Fig. 6 zeigt ein Bandscheibenimplantat mit den Knochenwachstumsstimulierenden Lamellen.
Fig. 7 beschreibt ein Beispiel einer Knochenverankerungseinrichtung.

### Weg(e) zur Ausführung der Erfindung

Die technischen Lösungen sind nachfolgend oft beispielhaft beschrieben. Dies soll als Mittel zur Erläuterung des zugrundeliegenden Gedankens aufgefasst und nicht als auf die jeweilige konkrete Darstellung beschränkt verstanden werden.

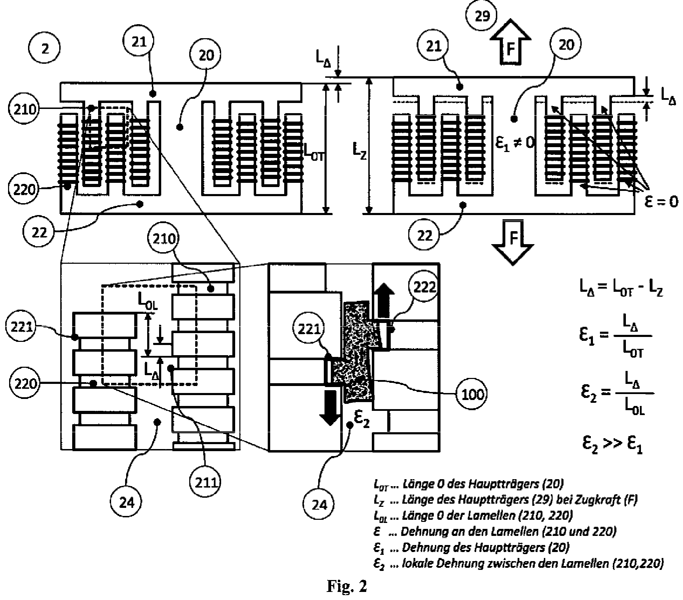

Das erfindungsgemäße Implantat 2 besteht aus mindestens einem Implantatträger 20 mit jeweils mindestens einem Paar Implantatgrenzflächen 21, 22 über die eine mechanische Belastung F in das Implantat eingeleitet wird, sowie mindestens einem Strukturträger 210 mit einer Struktur in Form von regelmäßigen oder unregelmäßigen Lamellen, Gittern, Ausformungen, Ausnehmungen, Bohrungen oder Kavitäten 211, 221. Die Belastung des Implantats kann dabei sowohl durch die alltäglichen Aktivitäten des Patienten, durch gezielte Belastungen in Form von Übungen (Physiotherapie) oder durch eine externe oder interne mechanische Belastung erfolgen. Falls zwei oder mehr Strukturträger eingesetzt werden, wird ein Strukturträger als erster Strukturträger 210 und der Zweite als zweiter Strukturträger 220 bezeichnet (Fig. 2). Die Grenzflächen 21, 22 besitzen jeweils ihnen zugeordnete und damit verbundene Strukturträger 210 und 220. Die Strukturträger 210, 220 sind weiterhin dahingehend charakterisiert, dass sie eine topologische Struktur 211 und 221 an bzw. in der Oberfläche aufweisen. Diese Struktur 211 und 221 kann beispielsweise durch Poren, Bohrungen, Nuten, Schlitze, Öffnungen, Texturen, Stege, Ausformungen, Aufrauhung, Beschichtungen oder sonstige Elemente die eine Struktur zur Generierung von Höhenunterschieden darstellen erzeugt werden. Sie haben die Aufgabe das Knochenmaterial, welches sich im Überlappungsbereich zwischen den Strukturträgern befindet bzw. dort bildet 24 mit einer lokalen Dehnung zu beaufschlagen und so das Knochenwachstum und damit die Osseointegration anzuregen.

Sind zwei oder mehr Grenzflächen 21 und 22 mit dem Implantatträger 20 verbunden, so können die Strukturträger 210 so angeordnet werden, dass sie sich bei Implantatbelastung aufeinander zu, voneinander weg, oder aneinander vorbei bewegen. Ein Spalt bzw. eine Einrichtung zur Sicherstellung eines Mindestabstands zwischen den Strukturträgern bzw. deren Oberflächen kann unphysiologisch hohe Dehnungen bzw. Quetschungen des Gewebes verhindern und damit der Zerstörung des sich bildenden Gewebes vorbeugen.

Wirkt eine Kraft oder ein Moment 29 über die Grenzflächen 21 und 22 auf das gesamte Implantat 2, verformt sich der Implantatträger 20, was durch die Dehnung ε1 charakterisiert ist. Die Strukturelemente, die am ersten 210 und zweiten 220 Strukturträger befestigt sind, erfahren selbst keine nennenswerte innere Verformung (ε=0). Die nachfolgend vereinfacht Lamellen genannten Strukturen der Strukturträger werden um den Gesamtbetrag der Implantatverformung (LΔ) relativ zu einander verschoben. Wird dieser Gesamtbetrag der Implantatverformung LΔ auf einen Abstand LOL der Vertiefungen zweier gegenüberliegender Lamellen bezogen, resultiert daraus eine lokale Dehnung ε2 im Zwischenraum 24, welche etwa im Verhältnis LΔ/ LOL um Größenordnungen über der eigentlichen Implantatdehnung ε1 liegt. Der Zwischenraum 24 ist mit Gewebe besiedelt oder mit Knochenmaterial befüllt und damit Ausgangspunkt für das Knochenwachstum. Durch die Wahl der Strukturgröße LOL ist die lokale Dehnung, die aus der Relativverschiebung der Lamellen 210 und 220 resultiert, in Kombination mit der Steifigkeit des Implantatträgers 20 praktisch beliebig einstellbar. Vorzugsweise wird die maximale Dehnung des Gewebes im Zwischenraum in Kombination mit der Strukturgröße LOL z.B. nach (Frost 1987) so gewählt, dass der lokale Dehnungsreiz an den Knochenzellen im Bereich für maximalen Knochenaufbau liegt. Die Verformung oder Deformation des Implantatträgers 20 erfolgt vorwiegend im elastischen, ermüdungsfrei ertragbaren Bereich. Obwohl der Implantatträger sich von seinen mechanischen Eigenschaften bzgl. seiner Elastizität nicht oder nur geringfügig von herkömmlichen rigiden Implantaten unterscheiden muss, sind die erfindungsgemäßen Ausgestaltungsformen in der Lage physiologisch für den Knochenaufbau wirksame Dehnungen zu generieren und auf das umliegende Gewebe zu übertragen.

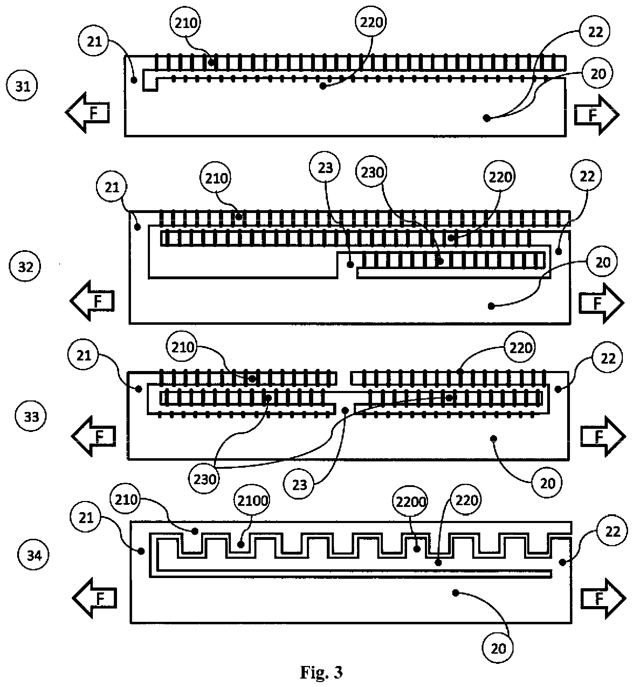

In weiterer Ausgestaltung der Erfindung kann die Anordnung der Lamellen variiert werden um verschiedene Dehnungsverläufe miteinander zu kombinieren (Fig. 3). Als erstes Beispiel 31 wird gezeigt, dass die Verwendung nur eines Strukturträgers bereits in der Lage ist, eine Vielzahl von lokal unterschiedlichen Dehnungen entlang der Implantatoberfläche zu erzeugen, wobei aufgrund der Mitverwendung des Implantatträgers ein nicht linearer Verlauf der lokalen Dehnung entsteht. Im dargestellten Beispiel nimmt die lokale Dehnung mit dem Abstand der Entfernung der Verbindung zwischen Implantatträger und Strukturträger proportional zu.

Im zweiten Beispiel 32 wird eine dritte Implantatgrenzfläche dargestellt, welche als mittlere Grenzfläche bezeichnet wird 23. Mit der Anordnung der Grenzflächen 21, 22 und 23 und den Strukturträgern 210, 220 und 230 können bestimmte Dehnungsbereiche eingestellt werden. Zum einen wird ein nichtlinearer Verlauf, wie in 31 gezeigt, zwischen dem Implantatträger 20 und dem Strukturträger 230 erzeugt. Darüber hinaus wird zwischen den Strukturträgern 220 und 230 eine gleichmäßige Dehnung generiert, welche im Zwischenraum zwischen 210 und 220 nochmals verdoppelt werden kann. So können beispielsweise drei Strukturträger so angeordnet sein, dass Dehnungsbereiche addiert werden können. Im Beispiel 33 können Dehnungsbereiche reduziert werden, indem die Verbindungsstelle des Strukturträgers beispielsweise mittig am Implantatträger 20 angebunden wird. In einem weiteren Beispiel 34 ist gezeigt, dass die Strukturträger 210 und 220 weitere strukturelle Ausführungen besitzen können, mit dem Ziel die Gesamtverformung (LΔ) großflächig auf das Knochengewebe zu übertragen. Die Kombination von Elementen mit unterschiedlicher Strukturgröße, z.B. eine lamellenartige Struktur mit einer Aufrauhung an der Oberfläche scheint hierfür besonders geeignet zu sein.

Zur Beschleunigung und weiteren Verbesserung der Knochenbildung beziehungsweise der Einheilung des erfindungsgemäßen Implantats können die Strukturträger 20, 21, 22, die Lamellen der Strukturträger 210, 220, 230 und insbesondere die Zwischenräume 24 zusätzlich ein osteoinduktives und/oder osteokonduktives Material enthalten oder damit beschichtet sein. Beispiele für solche Materialien sind Hydroxilapatit, (Tri-)Kalziumphosphat oder Proteine wie z.B. BMP oder RGD.

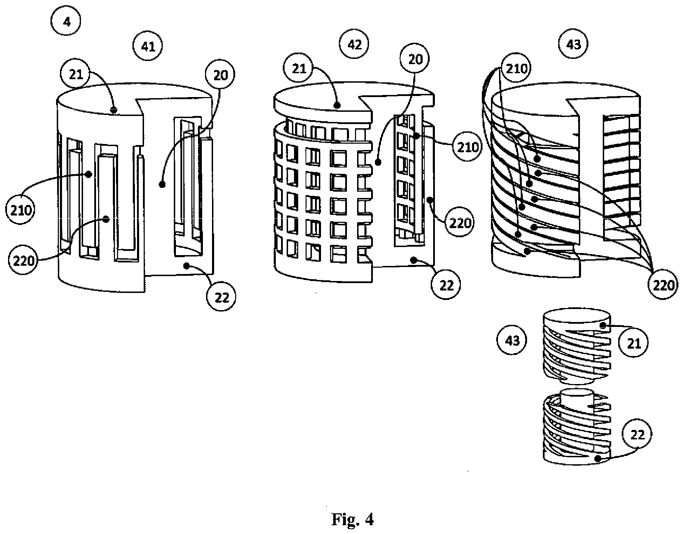

Ein Ausgestaltungsbeispiel eines Überbrückungselements zum Schließen großer Knochenfugen oder zur Fusion von Wirbelkörpern ist in Fig. 4 dargestellt. Hier wird am Beispiel eines Wirbelkörperersatzimplantats gezeigt, dass die Strukturträger 210 und 220 die Verformung des Implantatträgers 20 auf die Implantatoberfläche übertragen. Dabei können die Strukturträger 210 und 220 längs ineinandergreifend 41 oder radial überlappend 42 angeordnet sein, so dass die Strukturträger nur aus radialen Komponenten nur quer zur Belastungsrichtung bestehen. Beide Ausgestaltungsformen 41 und 42 sind zudem miteinander kombinierbar.

Eine weitere Ausgestaltung ist in 43 dargestellt, in der sich die Strukturträger 210, 220 - von deren Oberfläche aus gesehen - schräg zueinander bewegen, so dass die Relativbewegung der Strukturträger 210, 220 sowohl eine Scherungskomponente als auch eine axiale Verschiebungskomponente besitzen. Auch hier sind die beiden Strukturträger 210, 220 jeweils an nur einer Implantatgrenzfläche 21, 22 angebunden, so dass sich keine wesentliche Verformung der Strukturträger selbst ergibt, jedoch eine Relativverschiebung des Gewebes zwischen den Strukturträgern 210, 220. Betrachtet man eine mögliche, geringe Verformung der Strukturträger 210, 220 nicht, so entspricht die Relativverschiebung des Gewebes dabei der Verschiebung der Grenzflächen 21, 22. Des Weiteren können die Strukturträger 210, 220 auch kreuzweise angeordnet werden beispielsweise durch eine ineinandergreifende räumliche Gitterstruktur, was einer Mischform aus 42 mit 43 entspricht.

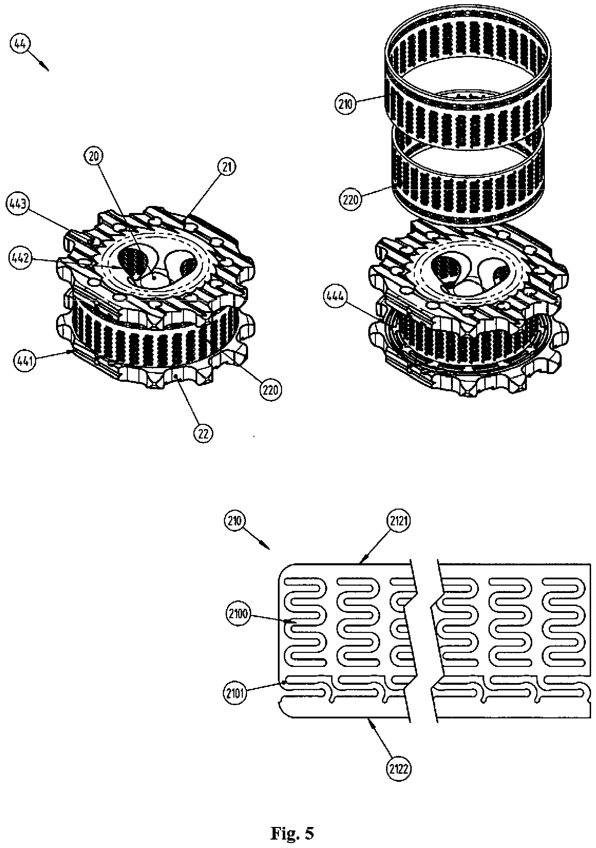

In Fig. 5 ist ein Zwischenwirbelimplantat 44 dargestellt, welches aus einem Implantatträger 20 und mehreren Lagen von Strukturträgern 210, 220 besteht. Der Implantatträger 20 ist dabei so ausgestaltet, dass er ein oder mehr Öffnungen 442 besitzt, welche zur Füllung mit Knochen(ersatz)material und/oder zum Nährstoffaustausch 443 dienen. Des Weiteren kann das Implantat eine Befestigungsmöglichkeit 441 zur Verbindung mit einem Implantationsinstrument besitzen. Das Implantat besitzt ferner eine obere 21 und eine untere 22 Grenzfläche an denen die Strukturträger 220 angebunden sind. Als eine Verbindungsmöglichkeit zu den Strukturträgern wird hier beispielhaft eine Schienenführung 444 gezeigt. In dieser Ausführungsform können die Strukturträger aus einem Blech hergestellt sein. Die Strukturträger 210, 220 werden zur Montage entlang der Schienenführung 444 in das Implantat 44 eingeschoben. Damit die Strukturträger 210, 220 mit ihren beiden Kanten 2121 und 2122 in der Schienenführung 444 des Implantatträgers 20 gehalten und so gegen ein Herausfallen gesichert sind, kann ein Verbund von Federn 2101 in den Platten vorgesehen werden. Die Federn übernehmen die Aufgabe, das als Strukturträger 210 ausgebildete Blech fest gegen eine der Grenzflächen zu drücken, so dass eine Bewegung der Grenzfläche mit einer Bewegung des Strukturträgers einhergeht. Selbstredend können die Strukturträger auch über eine alternative kraft-, form- oder stoffschlüssige Verbindung mit den Grenzflächen 21, 22 verbunden sein. Die in der Regel paarweise angeordneten Strukturträger werden dabei idealerweise abwechselnd mit jeweils einer der Grenzflächen 21 bzw. 22 fest verbunden.

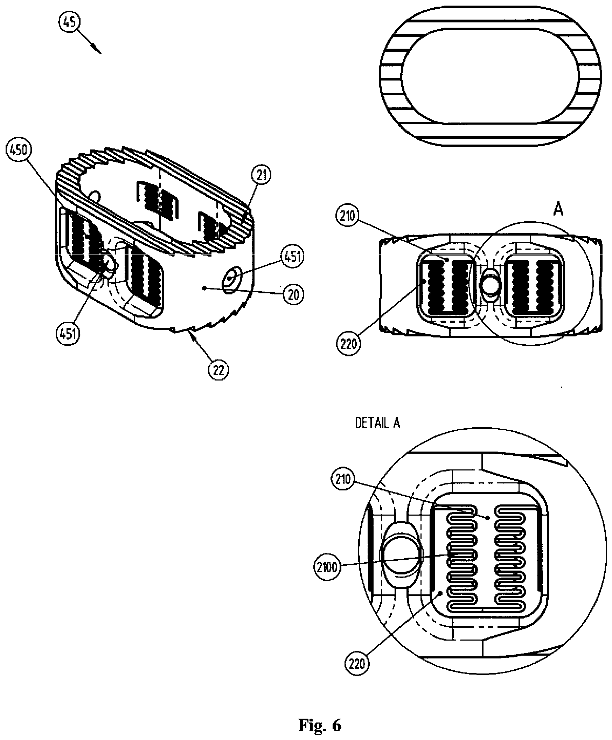

Ein weiteres Zwischenwirbelimplantat 45 mit einer an die Anatomie der Wirbelsäule angelehnten Form, welches eine obere 21 und eine untere 22 Grenzfläche besitzt, ist in Fig. 6 gezeigt. Das Zwischenwirbelimplantat besteht aus dem Implantatträger 20, welcher eine Aufnahme 451 für ein Implantationsinstrument beinhalten kann. In den Implantatträger 20 sind Aussparungen 450 eingelassen, die mit einem oder mehreren Strukturträgern 210, 220 ausgefüllt sind. Die Strukturträger 210 mit der Kennung sind an der oberen Grenzfläche 21 und die Strukturträger 220 mit der Kennung an die untere Grenzfläche 22 angebunden. Die in diesem Beispiel einteilig hergestellten Strukturträger 210, 220 sind durch mäanderförmige Ausschnitte 2100 in zwei Funktionsbereiche getrennt. Der Schnittspalt sowie die Mäanderform dient in diesem Fall der Erzeugung des Zwischenraums zur Übertragung der Dehnung auf das Gewebe.

Eine weitere vorteilhafte Ausgestaltungsform ergibt sich durch die integrale Verbindung eines Strukturträgers mit dem Implantatträger und Kombination mit mindestens einem zweiten Strukturträger der sich relativ zum Implantatträger bewegen kann. In diesem Fall erfolgt die Trennung zwischen lasttragenden Elementen und Elementen die einen lokalen Dehnungsreiz auf das Gewebe ausüben durch eine entsprechende Gestaltung von Ausschnitten, Öffnungen oder Verbindungselementen.

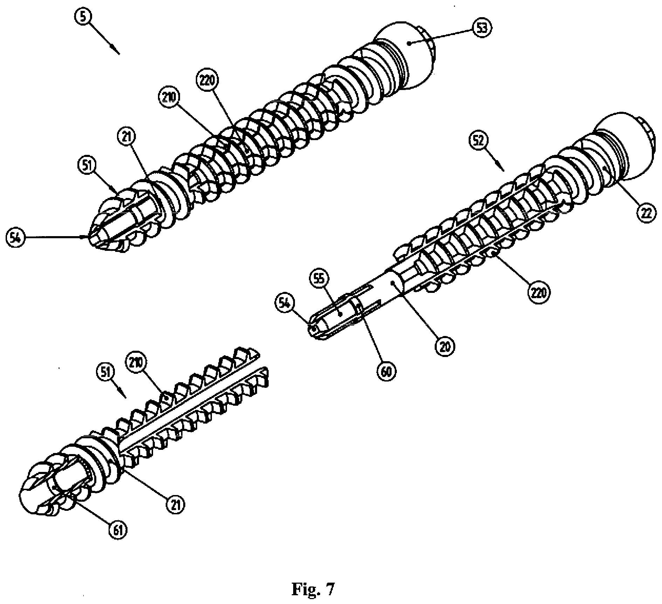

Eine beispielhafte Ausführung dieses Ansatzes ist anhand einer Knochenverankerungseinrichtung in Fig. 7 gezeigt, wie sie insbesondere als Pedikelschraube bei Wirbelsäulenimplantaten zum Einsatz kommt. Die Pedikelschraube 5 besteht aus einem distalen Schaftteil 51, einem proximalen Schaftteil 52, einem Schraubenkopf 53, einer optionalen Durchgangsöffnung 54 und einem Verbindungszapfen 55. Der Implantatträger 20 ist an der Verbindungsstelle mit dem Strukturträger 210 verbunden. Die Verbindungsstelle kann so ausgestaltet sein, dass sie starr, federelastisch oder wie im dargestellten Beispiel eine wegbegrenzte Relativbewegung zwischen Implantat- und Strukturträger zulässt. Hierzu befindet sich z.B. in der Bohrung 54 des Strukturträgers 21 eine Nut 61 die etwas breiter ist als der darin einrastende Bund 60 des Implantatträgers 20. Der Implantatträger 20 besitzt im dargestellten Beispiel außerdem einen Schraubenkopf 53 zur Verbindung mit weiteren Komponenten. Der distale Schaftteil 51 entspricht der ersten Grenzfläche 21, die wiederum durch die Gewindeflanken die Funktion des ersten Strukturträgers 210 übernimmt. Am proximalen Schaft 52 befindet sich die zweite Grenzfläche 22 mit ihrem zugeordneten Strukturträger 220 und den darauf befindlichen Gewindeflanken. Bei einer Zug- oder Biegebelastung der Schraube 5 kann nun eine Relativbewegung in Längsrichtung zwischen den Flanken der Strukturträger 210 und 220 stattfinden, begrenzt durch das Spiel zwischen Nut 61 und Bund 60. Durch die überlappende, ineinander gleitende Anordnung der beiden Strukturträger kann sich deren Relativbewegung nun auf den sich zwischen den Gewindeflanken befindliche Knochen übertragen und auf diesen einen Dehnungsreiz ausüben.

## Patentansprüche

1. Implantat in Form einer Knochenverankerungs- oder Verbindungseinrichtung bestehend aus einem Implantatträger (20), an dem mindestens ein Grenzflächenpaar (21, 22) zur Krafteinleitung und Anbindung an eine knöcherne Struktur ausgebildet ist, wobei mindestens zwei Strukturträger (210, 220, 230) vorgesehen sind, an denen jeweils Strukturelemente ausgebildet sind, **dadurch gekennzeichnet, dass** jeder der Grenzflächen (21, 22) mindestens ein Strukturträger (210, 220, 230) zugeordnet ist, dass mindestens Teilbereiche der Strukturträger (210, 220, 230) ineinander greifen oder überlappen und dass eine lokale Relativbewegung der Strukturträger (210, 220, 230) zueinander in etwa einer bei Belastung der Grenzflächen (21, 22) auftretenden Relativbewegung der Grenzflächen (21, 22) entspricht.

2. Implantat nach Anspruch 1, **dadurch gekennzeichnet, dass** die Strukturträger (210, 220, 230) eine Vielzahl kleinerer, nicht vollständig abgeschlossener Bereiche (24) definieren, die bei Implantatbelastung verformt werden.

3. Implantat nach Anspruch 2, **dadurch gekennzeichnet, dass** eine Teilung der Strukturelemente auf den Strukturträgern (210, 220, 230) und/oder ein Abstand zwischen den Strukturträgern (210, 220, 230) und eine Steifigkeit des Implantatträgers (20) derart auf eine erwartete mechanische Belastung des Implantats abgestimmt sind, dass die nicht vollständig abgeschlossenen Bereiche (24) bei einer Implantatbelastung eine Dehnung in einem für den Knochenaufbau günstigen Bereich erfahren.

4. Implantat nach Anspruch 2 oder 3, **dadurch gekennzeichnet, dass** die Strukturelemente selbsttragend ausgebildet sind.

5. Implantat nach einem der vorhergehenden Ansprüche, **dadurch gekennzeichnet, dass** die die Strukturelemente an den Strukturträgern (210, 220, 230) als Lamellen, Gitter, Ausformungen, Zähne, Ausnehmungen, Bohrungen, Poren, Texturen, Aufrauhungen, Beschichtungen oder Kavitäten ausgebildet sind.

6. Implantat nach Anspruch 5, **dadurch gekennzeichnet, dass** eine Oberfläche der Strukturträger (210, 220, 230) mit Strukturelementen versehen ist, die eine Strukturgröße im Bereich von 0.2*µ*m bis 100*µ*m aufweisen.

7. Implantat nach einem der vorhergehenden Ansprüche, **dadurch gekennzeichnet, dass** die Strukturträger (210, 220, 230) an einer der Grenzflächen (21, 22) mit einem Bereich (2101) mit niederer Steifigkeit und an einer anderen Grenzfläche (21, 22) mit einem Bereich mit höherer Steifigkeit angebunden sind.

8. Implantat nach einem der vorhergehenden Ansprüche, **dadurch gekennzeichnet, dass** die Strukturträger (210, 220, 230) abwechselnd mit je einer der Grenzflächen (21, 22) verbunden sind.

9. Implantat nach Anspruch 2, **dadurch gekennzeichnet, dass** die Strukturträger (210, 220, 230) mit einer Substanz zur Beschleunigung von Knochenbildung beschichtet oder angereichert sind und/oder dass die Bereiche (24) zwischen den Strukturträgern (210, 220, 230) mit einer Substanz zur Beschleunigung von Knochenbildung gefüllt sind.

10. Implantat nach Anspruch 2 oder 9, **dadurch gekennzeichnet, dass** Dehnungen in den Bereichen (24) in Folge der Implantatbelastung zwischen 100 und 15.000 *µ*m/m, und in der Einheilungsphase insbesondere zwischen 500 und 10.000 *µ*m/m liegen.

11. Implantat nach einem der vorherigen Ansprüche, **dadurch gekennzeichnet, dass** die maximale Relativbewegung der Strukturträger durch eine oder mehrere Wegbegrenzungen (60, 61) limitiert ist.

12. Implantat nach einem der vorherigen Ansprüche, **dadurch gekennzeichnet, dass** die maximale Relativbewegung der Strukturträger auf Werte zwischen 0,1*µ*m und 200*µ*m beschränkt ist.

## Claims

1. Implant in the form of a bone anchoring or connection device comprising an implant support (20) on which is formed at least one boundary surface pair (21, 22) for force initiation and connection to a bony structure, wherein at least two structural supports (210, 220, 230) are provided, on each of which structural elements are formed, **characterised in that** each of the boundary surfaces (21, 22) is assigned at least one structural support (210, 220, 230), that at least sections of the structural supports (210, 220, 230) engage in or overlap one another, and that a local movement of the structural supports (210, 220, 230) relative to one another corresponds roughly to a relative movement of the boundary surfaces (21, 22) occurring during loading of the boundary surfaces (21, 22).

2. Implant according to claim 1, **characterised in that** the structural supports (210, 220, 230) define a multiplicity of smaller, not completely closed areas (24), which are deformed under implant loading.

3. Implant according to claim 2, **characterised in that** a pitch of the structural elements on the structural supports (210, 220, 230) and/or a distance between the structural supports (210, 220, 230) and a stiffness of the implant support (20) are so matched to an expected mechanical loading of the implant that the not completely closed areas (24), during implant loading, undergo stretching in an area advantageous for bone structure.

4. Implant according to claim 2 or 3, **characterised in that** the structural elements are self-supporting.

5. Implant according to any of the preceding claims, **characterised in that** the structural elements on the structural supports (210, 220, 230) are in the form of lamellae, grids, mouldings, teeth, recesses, bores, pores, textures, roughening, coatings or cavities.

6. Implant according to claim 5, **characterised in that** a surface of the structural supports (210, 220, 230) is provided with structural elements which have a structure size in the range of 0.2 *µm* to 100*µ*m.

7. Implant according to any of the preceding claims, **characterised in that** the structural supports (210, 220, 230) are connected to one of the boundary surfaces (21, 22) by an area (2101) of low stiffness and to another boundary surface (21, 22) by an area of high stiffness.

8. Implant according to any of the preceding claims, **characterised in that** the structural supports (210, 220, 230) are connected alternately to one of the boundary surfaces (21, 22).

9. Implant according to claim 2, **characterised in that** the structural supports (210, 220, 230) are coated or enriched with a substance to accelerate bone formation, and/or that the areas (24) between the structural supports (210, 220, 230) are filled with a substance for the acceleration of bone formation.

10. Implant according to claim 2 or 9, **characterised in that** stretching in the areas (24) as a result of implant loading lie between 100 and 15,000 *µ*m/m, and in the healing phase in particular between 500 and 10,000 *µ*m/m.

11. Implant according to any of the preceding claims, **characterised in that** the maximum relative movement of the structural supports is limited by one or more travel limits (60, 61),

12. Implant according to any of the preceding claims, **characterised in that** the maximum relative movement of the structural supports is limited to values between 0.1 *µ*m and 200 *µ*m.

## Revendications

1. Implant se présentant sous la forme d'un système d'ancrage d'os ou de liaison d'os constitué d'un support d'implant (20), au niveau duquel au moins une paire de faces de limite (21, 22) est réalisée aux fins de l'application d'une force et aux fins du rattachement à une structure osseuse, dans lequel au moins deux supports de structure (210, 220, 230) sont prévus, au niveau desquels respectivement des éléments de structure sont réalisés, **caractérisé en ce qu'**au moins un support de structure (210, 220, 230) est associé à chacune des faces de limite (21, 22), qu'au moins des zones partielles des supports de structure (210, 220, 230) viennent en prise les unes avec les autres ou se chevauchent les unes les autres, et qu'un déplacement relatif local des supports de structure (210, 220, 230) les uns par rapport aux autres correspond approximativement à un déplacement relatif des faces de limite (21, 22) survenant lors d'une contrainte des faces de limite (21, 22).

2. Implant selon la revendication 1, **caractérisé en ce que** les supports de structure (210, 220, 230) définissent une pluralité de plus petites zones (24) non totalement fermées hermétiquement, qui sont déformées lors de la contrainte de l'implant.

3. Implant selon la revendication 2, **caractérisé en ce qu'**une division des éléments de structure sur les supports de structure (210, 220, 230) et/ou une distance entre les supports de structure (210, 220, 230) et une rigidité du support d'implant (20) sont adaptées de telle manière à une contrainte mécanique attendue de l'implant que les zones (24) non fermées totalement de manière hermétique subissent, lors d'une contrainte de l'implant, un allongement dans une plage favorable à la construction osseuse.

4. Implant selon la revendication 2 ou 3, **caractérisé en ce que** les éléments de structure sont réalisés de manière à être autoporteurs.

5. Implant selon l'une quelconque des revendications précédentes, **caractérisé en ce que** les éléments de structure au niveau des supports de structure (210, 220, 230) sont réalisés sous la forme de lamelles, de grilles, de parties déformées, de dents, d'évidements, d'alésages, de pores, de textures, de parties rugueuses, de revêtements ou de cavités.

6. Implant selon la revendication 5, **caractérisé en ce qu'**une surface des supports de structure (210, 220, 230) est pourvue d'éléments de structure, qui présentent une dimension de structure située dans la plage allant de 0,2 µm à 100 µm.

7. Implant selon l'une quelconque des revendications précédentes, **caractérisé en ce que** les supports de structure (210, 220, 230) sont attachés, au niveau d'une des faces de limite (21, 22), à une zone (2101) présentant une rigidité plus faible et, au niveau d'une autre face de limite (21, 22), à une zone présentant une rigidité plus élevée.

8. Implant selon l'une quelconque des revendications précédentes, **caractérisé en ce que** les supports de structure (210, 220, 230) sont reliés en alternance à respectivement une des faces de limite (21, 22).

9. Implant selon la revendication 2, **caractérisé en ce que** les supports de structure (210, 220, 230) sont revêtus ou enrichis d'une substance servant à accélérer la construction osseuse, et/ou que les zones (24) entre les supports de structure (210, 220, 230) sont remplies d'une substance servant à accélérer la construction osseuse.

10. Implant selon la revendication 2 ou 9, **caractérisé en ce que** des allongements se situent dans les plages (24) résultant de la contrainte de l'implant comprises entre 100 et 15 000 µm/m, et lors de la phase de cicatrisation en particulier comprises entre 500 et 10 000 µm/m.

11. Implant selon l'une quelconque des revendications précédentes, **caractérisé en ce que** le déplacement relatif maximal des supports de structure est limité par une ou plusieurs limitations de course (60, 61).

12. Implant selon l'une quelconque des revendications précédentes, **caractérisé en ce que** le déplacement relatif maximal des supports de structure est limité à des valeurs comprises entre 0,1 µm et 200 µm.
